# FASCICULE DE BREVET EUROPEEN

(11) **EP 2 596 124 B1**
(45) Date de publication et mention de la délivrance du brevet: **08.07.2015**
(21) Numéro de dépôt: 11736084.2
(22) Date de dépôt: 21.07.2011
(51) Int. Cl.: C12Q 1/37, C07K 7/06

(54) **NOUVEAUX SUBSTRATS A FLUORESCENCE REPRIMEE, LEUR PREPARATION ET LEUR UTILISATION POUR L' IDENTIFICATION, LA DETECTION ET LE DOSAGE DE LEGIONELLA PNEUMOPHILA**
NEUE SUBSTRATE MIT UNTERDRÜCKTER FLUORESZENZ, IHRE HERSTELLUNG UND VERWENDUNG ZUM IDENTIFIZIEREN, NACHWEISEN UND TESTEN VON LEGIONELLA PNEUMOPHILIA
NOVEL SUBSTRATES THE FLUORESCENCE OF WHICH IS SUPPRESSED, PREPARATION THEREOF, AND USE THEREOF FOR IDENTIFYING, DETECTING, AND ASSAYING LEGIONELLA PNEUMOPHILIA

(30) Priorité: 21.07.2010 FR 1055970
(43) Date de publication de la demande: 29.05.2013
(73) Titulaire: Pharmaleads, 75013 Paris (FR)
(72) Inventeur: PORAS, Hervé, 78870 Bailly (FR); OUIMET, Tanja, 75012 Paris (FR); FOURNIE-ZALUSKI, Marie-Claude, 75011 Paris (FR)
(74) Mandataire: Regimbeau
(86) Numéro de dépôt international: PCT/EP2011/062565
(87) Numéro de publication internationale: WO 2012/010668

(56) Documents cités:
- WO-A1-01/77369
- WO-A2-2005/121354
- MCINTYRE M ET AL: "RAPID IDENTIFICATION OF LEGIONELLA-PNEUMOPHILA ZINC METALLOPROTEASE USING CHROMOGENIC DETECTION", APMIS, WILEY INTERSCIENCE, DK, vol. 99, no. 4, 1 janvier 1991 (1991-01-01), pages 316-320, XP008043164, ISSN: 0903-4641, DOI: DOI:10.1111/J.1699-0463.1991.TB05155.X

### SEQUENCE LISTING

<110> PHARMALEADS
<120> NOUVEAUX SUBSTRATS A FLUORESCENCE REPRIMEE, LEUR PREPARATION ET LEUR UTILISATION POUR L'IDENTIFICATION, LA DETECTION ET LE DOSAGE DE LEGIONELLA PNEUMOPHILA
<130> 358686D28772
<150> FR 1055970
   <151> 2010-07-21
<160> 13
<170> PatentIn version 3.3
<210> 1
   <211> 8
   <212> PRT
   <213> artificial
<220>
   <223> peptide substrat
<220>
   <221> MOD_RES
   <222> (1)..(1)
   <223> ACETYLATION
<220>
   <221> misc_feature
   <222> (4)..(4)
   <223> X remplace la Pyrenylalanine
<220>
   <221> misc_feature
   <222> (5)..(5)
   <223> (3 NO2) Tyr
<220>
   <221> MOD_RES
   <222> (5)..(5)
   <223> AMIDATION
<400> 1
   Ser Lys Gly Xaa Tyr Gly Gly Lys
   1               5
<210> 2
   <211> 7
   <212> PRT
   <213> artificial
<220>
   <223> peptide substrat
<220>
   <221> MOD_RES
   <222> (1)..(1)
   <223> ACETYLATION
<220>
   <221> misc_feature
   <222> (4)..(4)
   <223> X remplace la Pyrenylalanine
<220>
   <221> misc_feature
   <222> (5)..(5)
   <223> (3 NO2) Tyr
<220>
   <221> MOD_RES
   <222> (7)..(7)
   <223> AMIDATION
<400> 2
   Ser Lys Gly Xaa Tyr Gly Lys
   1               5
<210> 3
   <211> 6
   <212> PRT
   <213> artificial
<220>
   <223> peptide substrat
<220>
   <221> MOD_RES
   <222> (1)..(1)
   <223> ACETYLATION
<220>
   <221> misc_feature
   <222> (4)..(4)
   <223> X remplace la Pyrenylalanine
<220>
   <221> misc_feature
   <222> (5)..(5)
   <223> (3 N02) Tyr
<220>
   <221> MOD_RES
   <222> (6)..(6)
   <223> AMIDATION
<400> 3
   Ser Lys Gly Xaa Tyr Lys
   1               5
<210> 4
   <211> 8
   <212> PRT
   <213> artificial
<220>
   <223> peptide substrat
<220>
   <221> MOD_RES
   <222> (1)..(1)
   <223> ACETYLATION
<220>
   <221> misc_feature
   <222> (4)..(4)
   <223> X remplace la Pyrenylalanine
<220>
   <221> misc_feature
   <222> (5)..(5)
   <223> (3 NO2) Tyr
<220>
   <221> MOD_RES
   <222> (8)..(8)
   <223> AMIDATION
<400> 4
   Ser Arg Gly Xaa Tyr Gly Gly Lys
   1 5
<210> 5
   <211> 7
   <212> PRT
   <213> artificial
<220>
   <223> peptide substrat
<220>
   <221> MOD_RES
   <222> (1)..(1)
   <223> ACETYLATION
<220>
   <221> misc_feature
   <222> (3)..(3)
   <223> X remplace la Pyrenylalanine
<220>
   <221> misc_feature
   <222> (4)..(4)
   <223> (3 N02) Tyr
<220>
   <221> MOD_RES
   <222> (7)..(7)
   <223> AMIDATION
<400> 5
   Arg Gly Xaa Tyr Gly Gly Lys
   1 5
<210> 6
   <211> 8
   <212> PRT
   <213> artificial
<220>
   <223> peptide substrat
<220>
   <221> MOD_RES
   <222> (1)..(1)
   <223> ACETYLATION
<220>
   <221> misc_feature
   <222> (2)..(2)
   <223> homo Arg
<220>
   <221> misc_feature
   <222> (4)..(4)
   <223> X remplace la Pyrenylalanine
<220>
   <221> misc_feature
   <222> (5)..(5)
   <223> (3 N02) Tyr
<220>
   <221> MOD_RES
   <222> (8)..(8)
   <223> AMIDATION
<400> 6
   Ser Arg Gly Xaa Tyr Gly Gly Lys
   1               5
<210> 7
   <211> 8
   <212> PRT
   <213> artificial
<220>
   <223> peptide substrat
<220>
   <221> MOD_RES
   <222> (1)..(1)
   <223> ACETYLATION
<220>
   <221> misc_feature
   <222> (2)..(2)
   <223> X remplace l'Ornithine
<220>
   <221> misc_feature
   <222> (4)..(4)
   <223> X remplace la Pyrenylalanine
<220>
   <221> misc_feature
   <222> (5)..(5)
   <223> (3 NO2) Tyr
<220>
   <221> MOD_RES
   <222> (8)..(8)
   <223> AMIDATION
<400> 7
   Ser Xaa Gly Xaa Tyr Gly Gly Lys
   1               5
<210> 8
   <211> 6
   <212> PRT
   <213> artificial
<220>
   <223> peptide substrat
<220>
   <221> MOD_RES
   <222> (1)..(1)
   <223> ACETYLATION
<220>
   <221> misc_feature
   <222> (4)..(4)
   <223> X remplace la Pyrenylalanine
<220>
   <221> misc_feature
   <222> (5)..(5)
   <223> (3 N02) Tyr
<220>
   <221> misc_feature
   <222> (6)..(6)
   <223> X remplace l'Ornithine
<220>
   <221> MOD_RES
   <222> (6)..(6)
   <223> AMIDATION
<400> 8
   Ser Lys Gly Xaa Tyr Xaa
   1               5
<210> 9
   <211> 6
   <212> PRT
   <213> artificial
<220>
   <223> peptide substrat
<220>
   <221> MOD_RES
   <222> (1)..(1)
   <223> ACETYLATION
<220>
   <221> misc_feature
   <222> (4)..(4)
   <223> X remplace la Pyrenylalanine
<220>
   <221> misc_feature
   <222> (5)..(5)
   <223> (3 NO2) Tyr
<220>
   <221> misc_feature
   <222> (6)..(6)
   <223> homo Lysine
<220>
   <221> MOD_RES
   <222> (6)..(6)
   <223> AMIDATION
<400> 9
   Ser Lys Gly Xaa Tyr Lys
   1               5
<210> 10
   <211> 6
   <212> PRT
   <213> artificial
<220>
   <223> peptide substrat
<220>
   <221> MOD_RES
   <222> (1)..(1)
   <223> ACETYLATION
<220>
   <221> misc_feature
   <222> (1)..(1)
   <223> homo Serine
<220>
   <221> misc_feature
   <222> (4)..(4)
   <223> X remplace la Pyrenylalanine
<220>
   <221> misc_feature
   <222> (5)..(5)
   <223> (3 NO2) Tyr
<220>
   <221> misc_feature
   <222> (6)..(6)
   <223> X remplace l'Ornithine
<220>
   <221> MOD_RES
   <222> (6)..(6)
   <223> AMIDATION
<400> 10
   Ser Lys Gly Xaa Tyr Xaa
   1               5
<210> 11
   <211> 6
   <212> PRT
   <213> artificial
<220>
   <223> peptide substrat
<220>
   <221> MOD_RES
   <222> (1)..(1)
   <223> ACETYLATION
<220>
   <221> misc_feature
   <222> (4)..(4)
   <223> X remplace la Pyrenylalanine
<220>
   <221> misc_feature
   <222> (5)..(5)
   <223> (3 N02) Tyr
<220>
   <221> misc_feature
   <222> (6)..(6)
   <223> X remplace l'Ornithine
<220>
   <221> MOD_RES
   <222> (6)..(6)
   <223> AMIDATION
<400> 11
   Ser Lys Gly Xaa Tyr Arg
   1               5
<210> 12
   <211> 6
   <212> PRT
   <213> artificial
<220>
   <223> peptide substrat
<220>
   <221> MOD_RES
   <222> (1)..(1)
   <223> ACETYLATION
<220>
   <221> misc_feature
   <222> (4)..(4)
   <223> X remplace la Pyrenylalanine
<220>
   <221> misc_feature
   <222> (5)..(5)
   <223> (3 NO2) Tyr
<220>
   <221> misc_feature
   <222> (6)..(6)
   <223> homo Arginine
<220>
   <221> MOD_RES
   <222> (6)..(6)
   <223> AMIDATION
<400> 12
   Ser Lys Gly Xaa Tyr Arg
   1               5
<210> 13
   <211> 6
   <212> PRT
   <213> artificial
<220>
   <223> peptide substrat
<220>
   <221> MOD_RES
   <222> (1)..(1)
   <223> ACETYLATION
<220>
   <221> MOD_RES
   <222> (4)..(4)
   <223> retro Abz Lys
<220>
   <221> MOD_RES
   <222> (5)..(5)
   <223> (3 N02) Tyr
<220>
   <221> MOD_RES
   <222> (6)..(6)
   <223> X remplace l'Ornithine
<220>
   <221> MOD_RES
   <222> (6)..(6)
   <223> AMIDATION
<400> 13
   Ser Lys Gly Lys Tyr Xaa
   1               5

## Description

La présente invention a pour objet un nouveau procédé de détection et de dosage des légionelles de l'espèce *Legionella pneumophila* dans tous les milieux potentiellement contaminés par cette bactérie.

Les légionelles sont des bactéries aquatiques que l'on rencontre dans les eaux naturelles (lacs, rivières et marais) et qui se développent particulièrement dans les eaux tièdes (entre 25 et 45°C). Ces bactéries sont responsables chez l'homme d'infections respiratoires aigues, les légionelloses (maladie du légionnaire, fièvre de Pontiac) ainsi que d'anomalies biologiques extra-pulmonaires non spécifiques. A ce jour, environ 50 espèces de *Legionella* ont été identifiées. L'espèce *L. pneumophila* est la plus répandue et la plus virulente pour l'homme. Parmi les 16 sérogroupes *de L. pneumophila* (représentant 90 à 95% des cas cliniques), le sérogroupe (SG) 1 est responsable de 84 % des infections humaines. L'infection apparait suite à l'inhalation d'aérosols chargés en légionelles, qui atteignent les alvéoles pulmonaires. Les bactéries vont alors se développer dans les macrophages alvéolaires puis dans le tissu pulmonaire. Les personnes atteintes par cette maladie sont, en général, des personnes âgées et/ou souffrantes de déficits immunitaires graves.

La maladie du légionnaire est considérée comme une infection opportuniste. En France, les données issues de la déclaration obligatoire font état de 1527 cas de légionellose déclarés en 2005, représentant une incidence de 2 cas pour 100000 habitants contre une estimation de 8000 à 18000 cas de légionellose chaque année aux États-Unis.

Les légionelles colonisent naturellement les réseaux d'eau d'alimentation. Elles sont présentes dans les eaux réchauffées des systèmes de conditionnement d'air, des tours aéroréfrigérantes (TAR), dans les réseaux d'eau chaude sanitaire (cumulus) où elles se multiplient de façon optimale entre 30° et 40°C. Elles peuvent contaminer des établissements comme les piscines, les équipements de stations thermales, les fontaines et, dans les hôpitaux, les humidificateurs, les respirateurs ou les nébulisateurs. En fait, les légionelles sont le plus souvent trouvées, en très forte quantité, dans les biofilms (Declerck et al., Curr. Microbiol., 2007, 55, 5, 435-440) associées à des protozoaires (amibes), qui, en les ingérant, permettent leur croissance intracellulaire et leur réplication. La détection des légionelles est soumise à réglementation dans les établissements à risques. Les normes actuelles pour la recherche et le dénombrement des légionelles sont basées sur l'emploi de techniques de mise en culture (norme internationale ISO 11731 et norme française AFNOR NF R90-431). Ces techniques sont très sensibles, mais longues à mettre en oeuvre (10 à 15 jours), délicates car les légionelles ont un taux de croissance faible et elles nécessitent un personnel qualifié pour l'établissement du diagnostic.

Des approches alternatives ont été développées au cours de ces dernières années :
1- La technique de PCR en temps réel (RT-PCR). Depuis 2006, une norme expérimentale (XP T 90-47) encadre cette méthode qui est rapide, sensible et spécifique. Cependant il n'existe pas de corrélation stricte entre UFC et Unité Génome (UG) accessible par PCR, ce qui pose un problème. D'autre part, cette technique nécessite un équipement de laboratoire particulier, un personnel qualifié et présente un coût élevé.
2- Les techniques de détection par immunochromatographie ou immunofluorescence. Ces approches immunologiques sont méthodologiquement plus simples, mais elles nécessitent des anticorps de large spécificité vis-à-vis des espèces recherchées et leur sensibilité varie beaucoup en fonction de la méthode de détection utilisée. Ces approches requièrent aussi un appareillage et un personnel adaptés et le prix de revient est élevé. Actuellement ces techniques ne sont pas utilisées en routine.
3- La méthode d'hybridation *in situ* avec détection fluorimétrique (FISH). Cette méthode récente (Declerck et Ollevier, Methods Mol. Biol., 2006, 345, 175-183) est sensible et rapide, mais elle nécessite, comme les précédentes, un matériel dédié d'un coût élevé et un personnel qualifié, ce qui rend la technique marginale.
4- L'ATPmétrie. Cette méthode permet de détecter la concentration de bactéries métaboliquement actives et représente un moyen d'alerte efficace. Des kits d'ATPmétrie sont disponibles à faible coût, mais ne sont pas spécifiques des légionelles.

Aucune de ces méthodes n'est à la fois spécifique, sensible, rapide, utilisable sur le terrain et d'un prix de revient raisonnable.

Il existe donc toujours un besoin d'une nouvelle méthode de détection et de dosage des légionelles présentant toutes les qualités précédemment définies.

Les présents inventeurs proposent une telle méthode en se basant sur la mesure de l'activité d'une protéase spécifique sécrétée par la bactérie.

Dès 1979, une protéase sécrétée par différentes souches de *L. pneumophila* a été mise en évidence (Baine et al., Journal of Clinical Microbiology, 1979, 9, 3, 453-456). Elle présente une activité protéolytique sur différentes protéines du sérum (Müller, Infect. Immun., 1980, 27, 51-53) ainsi que sur le collagène, la caséine et la gélatine (Thompson et al., Infect. Immun., 1981, 34, 299-302). C'est la protéine la plus abondante trouvée dans les surnageants de culture de *L. pneumophila,* d'où son nom *Major Secretory Protein,* **Msp.**

Enfin, la Msp fait partie des nombreux facteurs de virulence caractérisés dans la famille *Legionella :* elle présente des actions cytotoxiques et hémolytiques (Dowling et al., Microbiological Reviews, 1992, 56, 1, 32-60), en particulier chez le cobaye, en provoquant des hémorragies et des lésions nécrotiques (Conlan et al., J. Gen. Microbiol., 1986, 132, 1565-1574, Rosenfeld et al., FEMSMicrobiol. Lett., 1986, 37, 51-58). Il est intéressant de noter que ces propriétés cytotoxiques et hémolytiques sont directement reliées à l'activité protéasique de la Msp. En effet, la mutation du résidu Glu³⁷⁸, impliqué dans l'acte catalytique, conduit à une protéase inactive et non cytotoxique (Moffat et al., Mol. Microbiol., 1994, 12, 693-705).

Un substrat chromogénique, MeO-Suc-Arg-Pro-Tyr-pNA (S-2586), primitivement développé pour la caractérisation de l'α-chymotrypsine (Berdal et al., European Journal of Clinical Microbiology, 1982, 1, 1, 7-11), a été utilisé pour détecter la Msp dans différentes souches de légionelles (McIntyre et al., Acta Pathol. Microbiol. Immunol. Scand., 1991, 99, 4, 316-320), malgré le manque de sélectivité dudit substrat. Sur 283 souches testées de *Legionella pneumophila,* 282 ont dégradé le substrat. Sur 6 autres espèces de légionelles, seules 2 ont répondu positivement ainsi que certaines espèces de *Pseudomonas aeruginosa* (22 sur 40). Ces résultats préliminaires montrent donc une relative spécificité de la Msp de *L. pneumophila* par rapport à d'autres pathogènes pour le substrat S-2586 qui reste cependant peu sensible et pas suffisamment sélectif.

Compte tenu des importantes propriétés de la Msp, c'est à dire l'abondance de sa libération dans le milieu aqueux, son lien avec la pathogénicité de la bactérie et sa spécificité vis à vis des protéines excrétées par d'autres pathogènes, les présents inventeurs ont utilisé la Msp comme marqueur de la présence de *Legionella. pneumophila* dans différents réseaux d'eaux. Dans ce but, une méthode de détection et de quantification spécifique, sensible et rapide de cette protéase a été mise au point. Une corrélation statistiquement significative entre la quantité de Msp dosée et la quantité de *Legionella pneumophila* présente dans l'échantillon d'eau analysé a été établie.

La Msp a été primitivement purifiée à partir de surnageants de culture de *Legionella pneumophila* (Dreyfus et Iglewski, Infect. Immun., 1986, 51, 736-743) : c'est une métallopeptidase à zinc de 38 kDa, sous sa forme mature, de point isoélectrique 4,20 et dont le pH optimum de fonctionnement est compris entre 5,5 et 7,5. Le numéro d'accès de la Msp de *Legionella pneumophila* est P21347 dans Swissprot/UniProtKB. La séquence complète de la Msp de *Legionella pneumophila* est composée de 543 acides aminés répartis comme suit (résidus 1-24 : peptide signal, résidus 25-207 : séquence « propeptide », résidus 208-543 : métallo protéase à zinc).

Elle présente d'importantes homologies de séquences avec la Pseudolysine (*Pseudomonas aeruginosa elastase,* Black et al, J. Bacteriol., 1990, 172, 2608-2613) ainsi qu'avec la Thermolysine. Ces trois enzymes font partie de la famille M4 des métallopeptidases à zinc. Un alignement des séquences de la pseudolysine et de la Msp montre une homologie de 62,9% et permet de vérifier que la Msp possède les deux séquences consensus de cette famille à savoir les séquences ³⁷⁷HEVSH³⁸¹X₁₉ ⁴⁰¹ExxxD⁴⁰⁵dans lesquelles H³⁷⁷, H³⁹¹ et E⁴⁰¹ sont ligands du zinc et E³⁷⁸ est impliqué dans l'acte catalytique.

La présente invention a pour objet de proposer un nouveau test de dosage permettant de s'affranchir des problèmes rappelés plus haut qui sont liés aux tests existants. En particulier, ledit nouveau test de dosage selon l'invention est spécifique, sensible, rapide, utilisable sur le terrain et d'un prix de revient raisonnable.

La présente invention repose plus particulièrement sur l'utilisation par les inventeurs de peptides substrats sélectifs de la Msp qui permettent de détecter et de doser l'activité de l'enzyme. Lesdits peptides substrats comprennent un fluorophore Fluo, c'est-à-dire un acide aminé synthétique possédant une forte capacité de fluorescence de par la présence d'une chaine latérale fluorigénique. Avantageusement, cette fluorescence du reste Fluo est nulle ou substantiellement diminuée quand un répresseur Rep est situé dans la même molécule à proximité du fluorophore Fluo. En conséquence, la fluorescence naturelle du fluorophore Fluo ne peut se manifester qu'à partir du moment où le reste Fluo n'est plus soumis à l'effet répresseur du reste Rep : par exemple lorsque le peptide substrat est clivé par l'enzyme, ce qui entraîne la séparation physique des restes Fluo et Rep.

La présente invention a donc pour premier objet un peptide substrat sélectif de la Msp, de formule (I) :

(I) R-(X)ₙ-Fluo-Rep -(Gly)ₘ-Z-NH₂

dans laquelle,
- Fluo est un acide α-aminé de configuration (L), possédant sur sa chaîne latérale un groupement fluorigénique,
- Rep est un acide aminé de configuration (L) choisi parmi la (3-NO₂)Tyr et la (4-NO₂)Phe, étant entendu que lorsque Fluo est la pyrénylalanine, Rep est la (3-NO₂)Tyr,
- R est un groupement choisi parmi les groupements acétyle, succinyle et méthoxysuccinyle,
- X est un acide α-aminé de configuration (L) choisi dans le groupe constitué par : Gly, Ser, homo-Ser, Lys, homo-Lys, Arg, homo-Arg et Orn,
- Z est un acide α-aminé chargé positivement, et
- n et m sont deux entiers naturels, avec n compris entre 0 et 3 et m compris entre 0 et 2.

On entend par «acide α-aminé », au sens de la présente invention, tous les acides α-aminés naturels sous la forme L, ainsi que les acides α-aminés non naturels. Le terme « acides α-aminés naturels » représente entre autres les acides α-aminés suivants : la glycine (Gly), l'alanine (Ala), la valine (Val), la leucine (Leu), l'isoleucine (Ile), la sérine (Ser), la thréonine (Thr), la phénylalanine (Phe), la tyrosine (Tyr), le tryptophane (Trp), la cystéine (Cys), la méthionine (Met), la proline (Pro), l'acide aspartique (Asp), l'asparagine (Asn), la glutamine (Gln), l'acide glutamique (Glu), l'histidine (His), l'arginine (Arg), et la lysine (Lys). Les acides α-aminés non naturels au sens de l'invention comprennent des acides α-aminés non protéogéniques, tels que l'ornithine (Orn), l'homolysine (*homo-*Lys), l'homoarginine (*homo-*Arg), l'allylglycine, la tert-leucine, l'acide 2-amino-adipique, l'acide 1-amino-1-cyclobutanecarboxylique, l'acide 1-amino-1-cyclohexanecarboxylique, l'acide 1-amino-1-cyclopentanecarboxylique, l'acide 2-aminobutanoïque, l'acide 1-aminoindane-1-carboxylique, l'acide azétidine-2-carboxylique, l'acide (2*S*,4*R*)-4-benzyl-pyrrolidine-2-carboxylique, le γ-carboxyglutamate, la 2-cyclohexylalanine, la citrulline, la 5-hydroxylysine, l'acide 2,3-diamino-propionique, l'acide hippurique, l'homocyclohexylalanine, l'homophénylalanine, la 3-hydroxyproline, la 4-hydroxyproline, la 3-méthylhistidine, la 7-méthyllysine, l'acide indoline-2-carboxylique, l'α-méthyl-alanine, la norleucine, la norvaline, l'acide octahydroindole-2-carboxylique, la phénylglycine, l'acide 4-phényl-pyrrolidine-2-carboxylique, l'acide pipécolique, la propargylglycine, la 3-pyridinylalanine, la 4-pyridinylalanine, la sarcosine, l'acide 1,2,3,4-tétrahydroisoquinoline-1-carboxylique, ou l'acide 1,2,3,4-tétrahydroisoquinoline-3-carboxylique.

Les acides α-aminés non naturels au sens de l'invention comprennent également, par exemple tous les acides α-aminés naturels tels que définis ci-dessus, dans leur forme D. Les acides α-aminés non naturels selon l'invention comprennent aussi les acides α-aminés naturels, tels que définis plus haut, dans lesquels lesdits acides α-aminés comportent une chaine latérale modifiée pour inclure un groupement fluorigénique.

Le terme « chaîne latérale d'un acide aminé » représente le fragment porté par le carbone α d'un acide aminé. Par exemple, les chaînes latérales d'acides aminés naturels tels que la glycine, la valine, l'alanine et l'acide aspartique correspondent à l'atome d'hydrogène, aux groupes isopropyle, méthyle et CH₂COOH respectivement.

Par « groupement fluorigénique », on entend au sens de la présente invention un groupement chimique capable d'émettre un signal de fluorescence après excitation à une longueur d'onde correspondant à son maximum d'absorption.

De préférence, Fluo est choisi parmi les aminoacides suivants : la (L)-(1-pyrenyl)-alanine, la (L)-Nε(retroAbz)-Lys, la (L)-(7-methoxycoumarin-4-yl)-alanine, la (L)-((6,7-dimethoxy-coumarin-4-yl)-alanine, la (L)-Nβ(pyrenylacetyl)-Dap, la (L)-Nγ(pyrenylacetyl))-Dab, la (L)-Nδ-(pyrenylacetyl)-Orn, la (L)-Nε-(pyrenylacetyl)-Lys, la (L)-S-(1-pyrenemethyl)-Cys, la (L)-O-(1-pyrenemethyl)-Ser.

Préférentiellement, le reste Rep est la (3-NO₂)Tyr.

Dans un aspect préféré de l'invention, Z est un acide aminé chargé positivement de configuration (L). De manière encore plus préférée, Z est choisi parmi les (L)-Lys, (L)*-homo-*Lys, (L)-Orn, (L)-Arg, (L)-*homo*-Arg.

Les peptides sont avantageusement protégés en positions N-terminale par un groupement R= acétyle, succinyle ou méthoxysuccinyle, et en position C-terminale par un amide pour éviter toute dégradation non spécifique par des amino- ou carboxy-peptidases, qui pourraient être présentes dans le milieu.

De façon plus préférentielle, l'invention a pour objet un peptide substrat choisi dans le groupe consistant dans :
Composé **1**- Ac-Ser-Lys-Gly-**Pya-(3-NO₂)Tyr**-Gly-Gly-Lys-NH₂
Composé **2**- Ac-Ser-Lys-Gly-**Pya-(3-NO₂)Tyr**-Gly-Lys-NH₂
Composé **3**- Ac-Ser-Lys-Gly-**Pya-(3-NO₂)Tyr**-Lys-NH₂
Composé **4**- Ac-Ser-Arg-Gly-**Pya-(3-NO₂)Tyr**-Gly-Gly-Lys-NH₂
Composé **5**- Ac-Arg-Gly-**Pya-(3-NO₂)Tyr**-Gly-Gly-Lys-NH₂
Composé **6**- Ac-Ser-homo-Arg-Gly-**Pya-(3-NO₂)Tyr**-Gly-Gly-Lys-NH₂
Composé 7- Ac-Ser-Orn-Gly-**Pya-(3-NO₂)Tyr**-Gly-Gly-Lys-NH₂
Composé **8-** Ac-Ser-Lys-Gly-**Pya-(3-NO₂)Tyr**-Orn-NH₂
Composé **9**-Ac-Ser-Lys-Gly-**Pya-(3-NO₂)Tyr**-homo-Lys-NH₂
Composé **10** -Ac-homo-Ser-Lys-Gly-**Pya-(3-NO₂)Tyr**-Orn-NH₂
Composé **11** -Ac-Ser-Lys-Gly-**Pya-(3-NO₂)Tyr**-Arg-NH₂
Composé **12** -Ac-Ser-Lys-Gly-**Pya-(3-NO₂)Tyr**-homo-Arg-NH₂, et
Composé **13** -Ac-Ser-Lys-Gly-**(ε-Abz)Lys-(3-NO₂)Tyr**-Orn-NH₂.

De façon encore plus préférentielle, l'invention a pour objet un peptide substrat choisi dans le groupe constitué par les composés 1, 8 et 12 tels que définis ci-dessus.

La préparation des peptides substrats selon l'invention relève des compétences de l'homme du métier.

Les peptides substrats revendiqués peuvent être obtenus par des méthodes usuelles de synthèse en phase solide (voir par exemple Albericio, F. (2000). Solid-Phase Synthesis: A Practical Guide, 1ère ed., CRC Press). On peut ainsi utiliser, par exemple, la stratégie Boc ou la-stratégie Fmoc, toutes deux bien connues de l'homme du métier.

Les groupements protecteurs utilisables pour ces synthèses sont des groupements connus de l'homme du métier. Ces groupements protecteurs et leur utilisation sont décrits dans des ouvrages tels que par exemple Greene, "Protective Groups in Organic Synthesis", Wiley, New York, 2007 4ème édition ; Harrison et al. "Compendium of Synthetic Organic Methods", Vol. 1 à 8 (J. Wiley & sons, 1971 to 1996); Paul Lloyd-Williams, Fernando Albericio, Ernest Giralt, "Chemical Approaches to the Synthesis of Peptides and Proteins", CRC Press, 1997 ou Houben-Weyl, "Methods of Organic Chemistry, Synthesis of Peptides and Peptidomimetics", Vol E 22a, Vol E 22b, Vol E 22c, Vol E 22d., M. Goodmann Ed., Georg Thieme Verlag, 2002. Selon que ces groupements protecteurs sont portés par un atome d'azote, ils seront désignés en tant que groupements N-protecteurs. Il en est de même pour les groupements S-protecteurs, O-protecteurs, etc. Par exemple, un hydroxyle peut être protégé par un groupement trityle ou un acide carboxylique peut être protégé sous forme d'un ester tert-butylique. Si on fait une synthèse sur support solide, c'est la résine qui sert de groupement protecteur à la fonction carboxylique C-terminale.

Dans un aspect préféré de l'invention, la chimie utilisée correspond à la technologie Fmoc et la protection des chaînes latérales permettant leur clivage par l'acide trifluoroacétique (TFA), tel que décrit dans « Fmoc solid phase peptide Synthesis: a practical, approach W.C. Chan et P.D. White Eds. Oxford University Press, 2004 *».*

La réaction d'acylation pour conduire aux composés de formule (I) peut être réalisée dans les conditions usuelles connues de l'homme du métier.

Selon un aspect plus particulièrement préféré de l'invention, l'homme du métier met en oeuvre la stratégie Fmoc sur une résine paraméthylbenzhydrylamine (pMBHA), avec le mélange O-Benzotriazole-N,N,N',N'-tetramethyl-uronium-hexafluoro-phosphate (HBTU)/ Hydroxybenzotriazole (HOBt)/ N,N-Diisopropylethylamine (DIEA) comme agent de couplage. La déprotection des chaînes latérales est obtenue par action d'un mélange acide trifluoroacétique (TFA)/ triisopropylsilane (TIPS)/H₂O.

Les peptides sont purifiés par chromatographie en phase liquide à haute performance (HPLC). L'identité des peptides peut être confirmée par toute méthode connue de l'homme du métier telle que, par exemple, la spectrométrie de masse electrospray.

La fluorescence intrinsèque des peptides substrats de la formule (I) est très faible, compte tenu de la proximité spatiale entre le fluorophore et le reste répresseur Rep. L'apparition d'une fluorescence intense en présence de Msp est liée à la génération, suite à un clivage enzymatique, d'un métabolite de formule générale (II)

Ac-(X)ₙ-Fluo (II)

dans laquelle Ac, X et n et Fluo ont les mêmes définitions que ci-dessus.

Les peptides substrats de l'invention, tels qu'ils sont définis dans la formule (I), sont hautement spécifiques de la Msp. En effet, lesdits peptides de formule (I) ne sont pas clivés par d'autres métalloenzymes telles que la pseudolysine, la neprilysine, l'ACE, l'ECE etc.... En revanche, la présence d'un ou plusieurs aminoacides entre le reste Fluo et le reste Rep conduit à des substrats qui ont perdu leur sélectivité. Ils sont alors reconnus par d'autres peptidases comme la pseudolysine.

La nature des aminoacides en position X ou Z de la formule (I) influence aussi fortement la spécificité de la protéase. En effet, la présence à l'une ou l'autre de ces positions d'un aminoacide hydrophobe, tel que la norleucine, conduit à un composé qui n'est pas clivé par la Msp.

D'autre part, le choix du reste Rep est crucial. Un acide aminé plus volumineux que la (3-NO₂)-Tyr ou que la (4-NO₂)Phe, tel que la 3,5 dinitro-tyrosine ou la DNP-lysine, conduit à des composés qui ne sont pas substrats, mais qui peuvent être, éventuellement, inhibiteurs de la Msp. De plus l'exaltation de fluorescence observée après clivage des substrats contenant le couple pyrenylalanine/(3-NO₂)-Tyr est environ 2 fois plus importante qu'avec les substrats contenant le couple pyrenylalanine/(4-NO₂)Phe.

La spécificité des peptides de formule (I) pour la Msp permet de pouvoir affirmer que tout clivage d'un peptide de formule (I) est uniquement dû à l'action de la Msp. Ce clivage se traduit par l'émission de fluorescence, causée par la séparation physique du reste Fluo et du reste Rep. L'analyse par HPLC de la réaction montre qu'il n'y a que deux métabolites, donc un seul site de clivage et l'apparition de la fluorescence démontre que le clivage se situe, comme attendu, entre le fluorophore et le répresseur de fluorescence.

L'invention a donc aussi pour deuxième objet un procédé de détection de l'activité protéase de la Msp dans un échantillon d'une solution.

On entend par « solution » selon la présente invention toute solution dans laquelle on suspecte la présence de Msp. Comme Msp est une protéine sécrétée, la solution selon l'invention comprend ainsi tout milieu susceptible de contenir des *Legionella pneumophila.* Cela inclut donc aussi bien les cultures liquides de *Legionella. pneumophila* réalisées en laboratoire que les eaux chaudes sanitaires ou les eaux des tours aéroréfrigérantes, ou encore les lacs, les rivières, les étangs, les bassins et tout autre pièce d'eau naturelle ou artificielle. Une solution au sens de l'invention peut aussi comprendre la protéine Msp sous forme partiellement ou totalement purifiée. De telles solutions peuvent être obtenues en laboratoire lors d'étapes de purification (un exemple de purification permettant d'obtenir une telle solution de Msp purifiée est indiqué dans les exemples expérimentaux). Dans ce cadre, la détection de l'activité protéasique de Msp peut, par exemple, permettre de suivre la purification de la protéine.

Le procédé de détection de l'activité protéase de la Msp dans un échantillon d'une solution selon l'invention comprend les étapes de :
a) mise en contact dudit échantillon avec un composé de formule (I) tel que défini plus haut, et
b) détection d'une émission de fluorescence.

L'étape a) du procédé de l'invention peut être mise en oeuvre dans une large gamme de températures. Avantageusement, ladite température est comprise entre 20°C et 55°C ; préférentiellement, elle est comprise entre 25°C et 45°C ; plus préférentiellement, elle est comprise entre 30°C et 40°C ; encore plus préférentiellement, elle est comprise entre 35°C et 38°C. Selon le mode de réalisation le plus préféré de l'invention, elle est égale à 37°C.

Une molécule fluorescente possède la propriété d'absorber de l'énergie à une longueur d'onde d'excitation définie et de la restituer rapidement sous forme de signal fluorescent, à une longueur d'onde d'émission définie. L'émission de fluorescence de l'étape b) du procédé est détectée à une longueur d'onde d'émission spécifique qui dépend du groupement fluorigénique porté par le reste Fluo. L'adaptation de ladite longueur d'onde d'émission en fonction dudit groupement fluorigénique fait partie des capacités de l'homme du métier. Celui-ci, par exemple, sait que le spectre d'émission de la pyrénylalanine présente deux maximas à 377 nm et 397 nm. Il peut ainsi utiliser plusieurs longueurs d'ondes autour de ces valeurs pour mesurer l'émission de fluorescence quand le reste Fluo comprend une pyrénylalanine. Avantageusement, la longueur d'onde d'émission est comprise entre 365 et 405 nm pour la pyrénylalanine. Préférentiellement, ladite longueur d'onde est comprise entre 370 et 400 nm. Encore plus préférentiellement, elle est égale à 377 nm. La longueur d'onde d'émission est avantageusement de 410 nm pour l'aminobenzoyle et 420 nm pour les dérivés méthoxycoumarinyles.

Tout comme la longueur d'onde d'émission, la longueur d'onde d'excitation est spécifique du groupement fluorigénique porté par le reste Fluo. Encore une fois, l'homme du métier est parfaitement capable d'adapter la longueur d'onde d'excitation audit groupement fluorigénique. Avantageusement, la longueur d'onde d'excitation est 340 nm pour la pyrénylalanine, 310 nm pour l'aminobenzoyle et 335 nm pour les dérivés méthoxycoumarinyles.

L'émission de fluorescence produite par le clivage d'un peptide de l'invention par la Msp peut être détectée à l'aide de tout moyen connu de l'homme du métier comme convenant à cet effet. On mentionnera en particulier parmi lesdits moyens les fluorimètres. De nombreux types de fluorimètres existent et l'homme du métier saura identifier les modèles qui conviennent le mieux en fonction de ses besoins. On utilisera de façon préférée un spectrofluorimètre, lequel est utilisable sur toute la gamme des longueurs d'onde (200-800 nm) aussi bien en excitation qu'en émission. Le fluorimètre possède l'avantage de pouvoir mesurer l'intensité de la fluorescence émise. De façon avantageuse, la mesure de la fluorescence est répétée à intervalles réguliers ou non au cours du temps, afin de déterminer par exemple les paramètres cinétiques de la réaction de clivage. Encore plus avantageusement, la réaction de clivage est suivie de manière continue au cours du temps.

Les inventeurs ont ainsi montré que la réponse fluorimétrique varie de façon linéaire en fonction de la concentration en protéase. Il est donc possible, à partir d'une valeur de fluorescence, de déduire facilement à quelle concentration de Msp ladite valeur de fluorescence correspond. On peut, par exemple, selon une technique bien connue de l'homme du métier, établir une gamme étalon avec des quantités connues de Msp pour déterminer la quantité de Msp présente dans l'échantillon.

La présente invention a donc pour troisième objet un procédé de dosage de la Msp dans un échantillon d'une solution comprenant les étapes de :
a) détecter l'activité protéase de la Msp selon le procédé décrit plus haut,
b) mesurer l'émission de fluorescence, et
c) déduire de la valeur de l'émission de fluorescence la quantité de Msp présente dans l'échantillon.

Il peut exister des cas où la quantité de Msp à détecter est très faible. Dans d'autres cas, la solution contenant la Msp peut aussi contenir des composés inhibant l'activité protéasique de Msp. Dans ces conditions, il peut être avantageux de concentrer l'enzyme avant de mesurer l'activité de l'enzyme pour accroitre la sensibilité du test. On peut ainsi filtrer et concentrer l'échantillon, par exemple en passant ledit échantillon sur un filtre Centricon. De préférence, cette étape de concentration permettra d'éliminer toutes les molécules dont le poids moléculaire est inférieur ou égal à 25 kDa. On peut aussi procéder à un enrichissement sélectif en protéine Msp, par exemple en utilisant un anticorps anti-Msp n'affectant pas l'activité catalytique de cette protéase (dans une colonne d'affinité ou dans une réaction d'immunoprécipitation) ou une colonne d'inhibiteur. L'enrichissement peut également être non sélectif, par exemple par concentration sur billes magnétiques. Le procédé de dosage de la Msp selon l'invention peut donc comprendre une étape supplémentaire de concentration de l'enzyme. Avantageusement, cette étape est réalisée avant l'étape a).

Dans un quatrième aspect, l'invention porte sur un procédé de dosage de *Legionella pneumophila* dans un échantillon d'une solution. En effet, les inventeurs ont démontré qu'il existait une relation linéaire entre la quantité de Msp détectée et la quantité de *Legionella pneumophila* présente dans ce même échantillon.

L'invention a donc aussi pour objet un procédé de dosage des *Legionella pneumophila* dans un échantillon d'une solution, comprenant les étapes de :
a) doser la Msp dans ledit échantillon selon le procédé décrit plus haut, et
b) en déduire la quantité de *Legionella pneumophila.*

Les composés de formule (I) sont spécialement utiles pour identifier, détecter et doser la présence de *Legionella pneumophila* dans des réseaux d'eau chaude sanitaire ou de TARs.

Différentes eaux de TAR plus ou moins contaminées par *L. pneumophila* ont été testées dans le but de déterminer la présence de Msp par le dosage fluorimétrique mis au point dans la revendication précédente. Parmi tous les essais effectués, aucun faux positif n'a été observé et la présence de Msp détectée dans des eaux dont la contamination était faible (inférieure à 1000 UG/L).

La présente invention propose également des kits pour la détection et le dosage de *Legionella pneumophila,* ledit kit contenant au moins un peptide de formule (I) tel que décrit ci-dessus. En outre, ledit kit comprend avantageusement les réactifs nécessaires à la mesure de l'activité enzymatique. Les kits selon l'invention sont utilisables au laboratoire ou sur le terrain.

Les figures et exemples ci-après sont présentés à titre illustratif et non limitatif de la présente invention.

### Légendes des figures

Figure 1
   Spécificité du substrat fluorigénique envers la Msp : Clivage comparatif du **composé 8** (10 µM) par 10 ng/mL de Msp et de pseudolysine, pepsine, papaïne, neprilysine (NEP), enzyme de conversion de l'endothéline-1 (ECE-1) et 2 (ECE-2), la protéase de HIV (HIV P.) ainsi que par la trypsine. Les deltas de fluorescence (U.A.) sont représentés après 300 minutes d'incubation à 37°C dans 50 mM HEPES pH 7.
Figure 2
   Comparaison de la dégradation du **composé 1** et de 2 composés **EF** et **EL** de formule respective Ac-(X)ₙ-Pya-EF-(3-NO₂)Tyr-(Gly)ₘ-Z-NH₂ et Ac-(X)ₙ-Pya-EL-(3-NO₂)Tyr-(Gly)ₘ-Z-NH₂ par la Msp et la Pseudolysine. Les substrats (10 µM) sont incubés pendant 120 minutes à 37°C avec 10 ng/mL de Msp purifiée ou de pseudolysine dans 50 mM HEPES pH 7.
Figure 3
   Comparaison de la dégradation du **composé 1** et des composés **Nop** de formule Ac-(X)ₙ-Pya-(NO₂)Phe-(Gly)ₘ-Z-NH₂ et **3,5-NO₂ Tyr** de formule Ac-(X)ₙ-Pya-(3, 5-(NO₂)₂)Tyr-(Gly)ₘ-Z-NH₂ par la Msp. Les substrats (10µM) sont incubés pendant 300 minutes à 37°C avec 2 ng/mL de Msp purifiée dans 50 mM HEPES pH 7.
Figure 4
   Comparaison de la dégradation (évolution de la fluorescence) en fonction du temps du **composé 1** et du composé **Nle** de formule Ac-S-Nle-G-Pya-(3-NO₂)Tyr-(Gly)ₘ-Z-NH₂ par la Msp. Les substrats (10 µM) sont incubés pendant 240 minutes à 37°C avec 10 ng/mL de Msp purifiée dans 50 mM HEPES pH 7. Les lectures sont effectuées au Berthold LS970B à λex = 340nm, λem = 405 nm et l'énergie de la lampe à 10000.
Figure 5
   Spectre d'émission de fluorescence du **composé 1** (c: 100µM) (en triangle) et de son métabolite fluorescent (c: 1µM) (en carré).
   Les peptides sont en solution dans le tampon HEPES 50 mM pH 7.
   En abscisse : les longueurs d'onde exprimées en nm. En ordonnée : l'intensité de fluorescence exprimée en unité arbitraire de fluorescence (AU).
   Le spectre est enregistré sur un appareil Perkin Elmer LS50B. (λex =343 nm, fente d'excitation : 15, fente d'émission : 2,5).
   Rapport des intensités de fluorescence à 377 nm : I(métabolite)/I(substrat) = 20000.
Figure 6
   Détection de la Msp dans le tampon HEPES 50 mM pH 7.
   6a) Évolution de la fluorescence (exprimé en Δ de fluorescence) en fonction du temps (min). L'essai est réalisé en microplaque 96 puits à 37°C, dans un volume final de 100 µL, en présence d'une concentration en **composé 1** de 10 µM et de concentrations en Msp variant de 0,1 à 30 ng/mL.
   6b) Linéarisation de la variation de fluorescence en fonction de la concentration en Msp (pg dans 100 µL) variant de 0,1 à 10 ng/mL après une incubation de 90 min. Des résultats analogues sont obtenus à 30, 60 et 120 min.
Figure 7
   7a) Relation entre la quantité de Msp présente dans un surnageant de culture (reflétée par la variation de fluorescence du **composé 1**) et la quantité de *Legionella pneumophila* dénombrée dans un milieu de culture. L'essai est réalisé en microplaque à 37°C, dans un volume final de 100 µL, en présence d'une concentration en composé 1 de 10 µM et de dilutions sérielles d'un surnageant de culture de 1/1000 à 1/20000.
   7b) Linéarisation de la variation de fluorescence en fonction du dénombrement (UFC/mL) en *Legionella pneumophila* après une incubation de 90 min. Le dénombrement a été effectué suivant le protocole de la norme AFNOR NF R90-431.
Figure 8
   8a) Variation de fluorescence induite par clivage des composés **1**, **8 et 12** par la Msp purifiée en fonction du temps.
   L'essai est réalisé dans le tampon HEPES 50mM pH 7 avec une concentration en substrat de 10 µM et une concentration en Msp de 10 ng/mL. Les lectures sont effectuées au Berthold LS970B à λex= 340 nm, λem = 405 nm et l'énergie de la lampe à 10000.
   8b) Détermination des paramètres cinétiques du **composé 1**.
   Variation de la vitesse de dégradation par la Msp (2 ng/ml) après une incubation 30 min à 37°C en fonction concentration du **composé 1** (µM).
   L'essai est réalisé dans le tampon HEPES 50 mM pH 7 avec une concentration en substrat de 10 µM et une concentration en Msp de 10 ng/mL. Les lectures sont effectuées au Berthold LS970B à λex = 340 nm, λem = 405 nm et l'énergie de la lampe à 10000.

### Exemple 1 : Préparation des substrats

Les peptides substrats (I) et leurs métabolites fluorescents (II) sont préparés en phase solide sur un synthétiseur automatique en utilisant la stratégie Fmoc et le protocole classique de couplage HBTU/HOBt/DIEA sur une résine MBHA pour les substrats et sur une résine HMP pour les métabolites fluorescents. Les chaînes latérales fonctionnalisées sont protégées sous forme de t-butyle éthers (Ser ou homo-Ser), de Pmc (Arg, homo-Arg) ou de Boc (Lys, homo-Lys, Orn) comme décrit dans « Fmoc solid phase peptide synthesis : A practical approach. W.C.Chan and P.D. White Eds. Oxford University Press, 2004 *».*

Les couplages sont effectués dans la N-méthyl-pyrrolidone (NMP) avec 10 équivalents d'acides aminés. Le fluorophore est introduit par couplage dans une seringue en présence de BOP/DIEA. L'acide aminé N-terminal est introduit directement sous forme de dérivé N-acétylé. La déprotection des chaînes latérales est obtenue en 2h par action d'un mélange TFA/TIPS/H₂O: (95/2,5/2,5) à température ambiante.

Les peptides sont purifiés par HPLC semi-préparative sur un appareil Waters 600 équipé d'un détecteur UV 2487, sur une colonne ACE C18 100Ǻ, 5 µm, 250 x 20mm ou Atlantis T3, 3.5 µm, 100 x 20 mm avec comme système d'élution un mélange CH₃CN (0,1% TFA)/ H₂O (0,1% TFA) en proportion variable. Leur pureté est vérifiée en HPLC analytique, sur une colonne ACE 100Ǻ, 5 µm, ou Atlantis T3, 3.5 µm, 100 x 4,6mm sur une HPLC Shimadzu Prominence avec un spectromètre UV pour la détection.

Les peptides sont analysés par spectrométrie de masse par Electrospray en mode positif sur une LCMS Agilent série 1200 détection simple Quad.

Les exemples suivants illustrent la présente demande, sans la limiter :
Composé **1-** Ac-Ser-Lys-Gly-**Pya-(3-NO₂)Tyr**-Gly-Gly-Lys-NH₂
   HPLC ACE 100Ǻ, 5 µm, C18 250 x 4,6mm CH₃CN (0,1% TFA)/ H₂O (0,1% TFA) 10 à 90 en 30 min ; Rt: 13,69 min. Masse ESI(+) : [(M+2H)/2]⁺ = 527,4
Composé **2-** Ac-Ser-Lys-Gly-**Pya-(3-NO₂)Tyr**-Gly-Lys-NH₂
   HPLC ACE 100Ǻ, 5 µm, C18 250 x 4,6mm CH₃CN (0,1% TFA)/ H₂O (0,1% TFA) 10 à 90 en 30 min ; Rt: 13,09 min. Masse ESI(+): [(M+2H)/2]⁺ = 498,9
Composé **3-** Ac-Ser-Lys-Gly-**Pya-(3-NO₂)Tyr**-Lys-NH₂
   HPLC ACE 100Ǻ, 5 µm, C18 250 x 4,6mm CH₃CN (0,1% TFA)/ H₂O (0,1% TFA)30/70 ; Rt: 12,87 min. Masse ESI(+): [(M+2H)/2]⁺ = 470,3
Composé **4-** Ac-Ser-Arg-Gly-**Pya-(3-NO₂)Tyr**-Gly-Gly-LyS-NH₂
   HPLC ACE 100Ǻ, 5 µm, C18 250 x 4,6mm CH₃CN (0,1% TFA)/ H₂O (0,1% TFA) 0 à 30% en 20 min puis 30% 10 min ; Rt: 29,83 min. Masse ESI(+): [(M+2H)/2]⁺ = 541,4 Composé **5-** Ac-Arg-Gly-**Pya-(3-NO₂)Tyr**-Gly-Gly-Lys-NH₂
   HPLC Atlantis T3, 3.5 µm, 100 x 4,6mm CH₃CN (0,1% TFA)/H₂O (0,1% TFA) 28/72 ; Rt : 15,40 min. Masse ESI(+): [(M+2H)/2]⁺ = 497,8
Composé **6-** Ac-Ser-homo-Arg-Gly-**Pya-(3-NO₂)**Tyr-Gly-Gly-Lys-NH₂
   HPLC Atlantis T3, 3.5 µm, 100 x 4,6mm CH₃CN (0,1% TFA)/ H₂O (0,1% TFA) 30/70 ; Rt : 13,21 min. Masse ESI(+): [(M+2H)/2]⁺ = 548,5
Composé **7-** Ac-Ser-Orn-Gly-**Pya-(3-NO₂)Tyr**-Gly-Gly-Lys-NH₂
   HPLC ACE 100Ǻ, 5 µm, C18 250 x 4,6mm CH₃CN (0,1% TFA)/ H₂O (0,1% TFA) 25/75% ; Rt: 41,62 min. Masse ESI(+): [(M+2H)/2]⁺ = 520,5
Composé **8-** Ac-Ser-Lys-Gly-**Pya-(3-NO₂)Tyr**-Orn-NH₂
   HPLC Atlantis T3, 3.5 µm, 100 x 4,6 mm CH₃CN (0,1% TFA)/ H₂O (0,1% TFA) 10 à 90% en 15 min ; Rt : 9,02 min. Masse ESI(+): [(M+2H)/2]⁺ = 477,8
Composé **9**-Ac-Ser-Lys-Gly-**Pya-(3-NO₂)Tyr**-homo-Lys-NH₂
   HPLC Atlantis T3, 3.5 µm, 100 x 4,6 mm CH₃CN (0,1% TFA)/ H₂O (0,1% TFA) 10 à 90% en 15 min ; Rt : 8,97 min. Masse ESI(+): [(M+2H)/2]⁺ = 463,5
Composé **10** -Ac-homo-Ser-Lys-Gly-**Pya-(3-NO₂)Tyr**-Orn-NH₂
   HPLC ACE 100Ǻ, 5 µm, C18 250 x 4,6 mm CH₃CN (0,1% TFA)/ H₂O (0,1% TFA)30/70 ; Rt: 13,70 min. Masse ESI(+): [(M+2H)/2]⁺ = 470,5
Composé **11** -Ac-Ser-Lys-Gly-**Pya-(3-NO₂)Tyr**-Arg-NH₂
   HPLC ACE 100Ǻ, 5 µm, C18 250 x 4,6 mm CH₃CN (0,1% TFA)/ H₂O (0,1% TFA) 30/70; Rt: 10,28 min. Masse ESI(+): [(M+2H)/2]⁺ = 484,0
Composé **12** -Ac-Ser-Lys-Gly-**Pya-(3-NO₂)Tyr**-homo-Arg-NH₂
   HPLC ACE 100Ǻ, 5 µm, C18 250 x 4,6 mm CH₃CN (0,1% TFA)/ H₂O (0,1% TFA) 30/70; Rt: 11,25 min. Masse ESI(+): [(M+2H)/2]⁺ = 491,5
Composé **13** -Ac-Ser-Lys-Gly-**(ε-Abz)Lys-(3-NO₂)Tyr**-Orn-NH₂
   Atlantis T3, 3.5 µm, 100 x 4,6 mm CH₃CN (0,1% TFA)/ H₂O (0,1% TFA) 15/85; Rt : 7,24 min. Masse ESI(+): [(M+2H)/2]⁺ = 451,6

### Exemple 2 Comparaison de la fluorescence émise par les substrats et leurs métabolites fluorescents.

Les longueurs d'onde d'excitation des substrats précédents sont :
λex = 340 nm lorsque le fluorophore est une pyrénylalanine, λex = 310 nm pour groupement aminobenzoyle et λex =335nm pour les dérivés méthoxycoumarinyles.

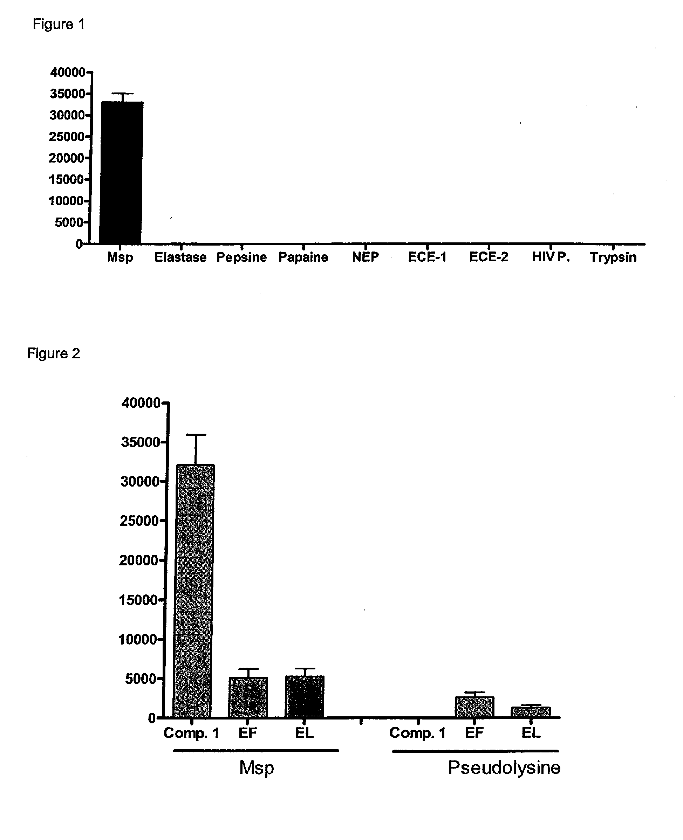

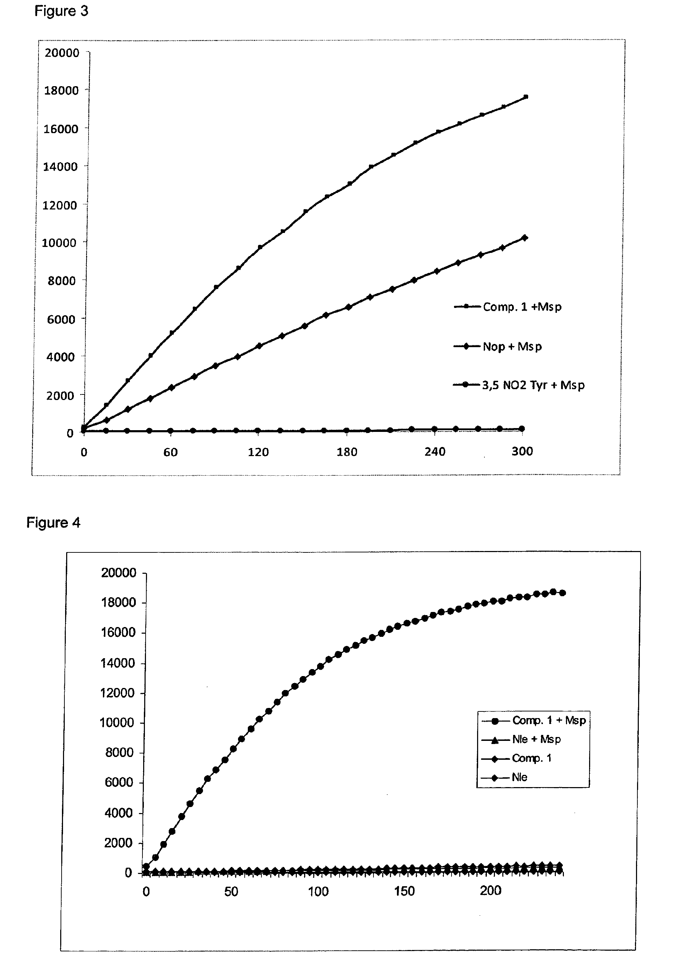

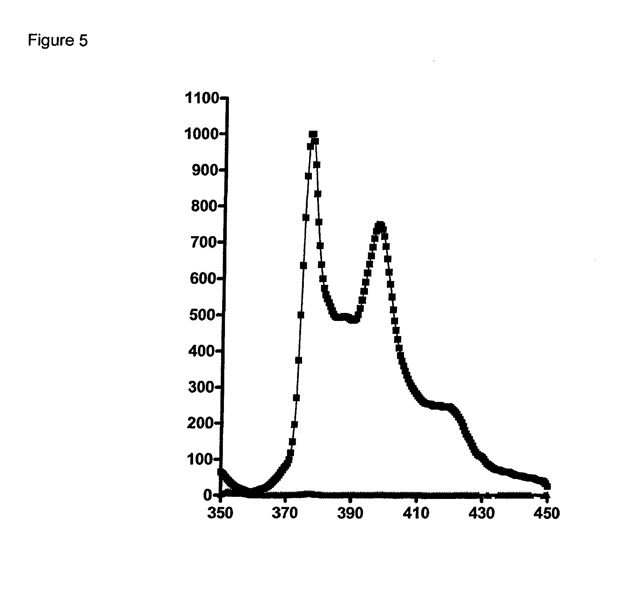

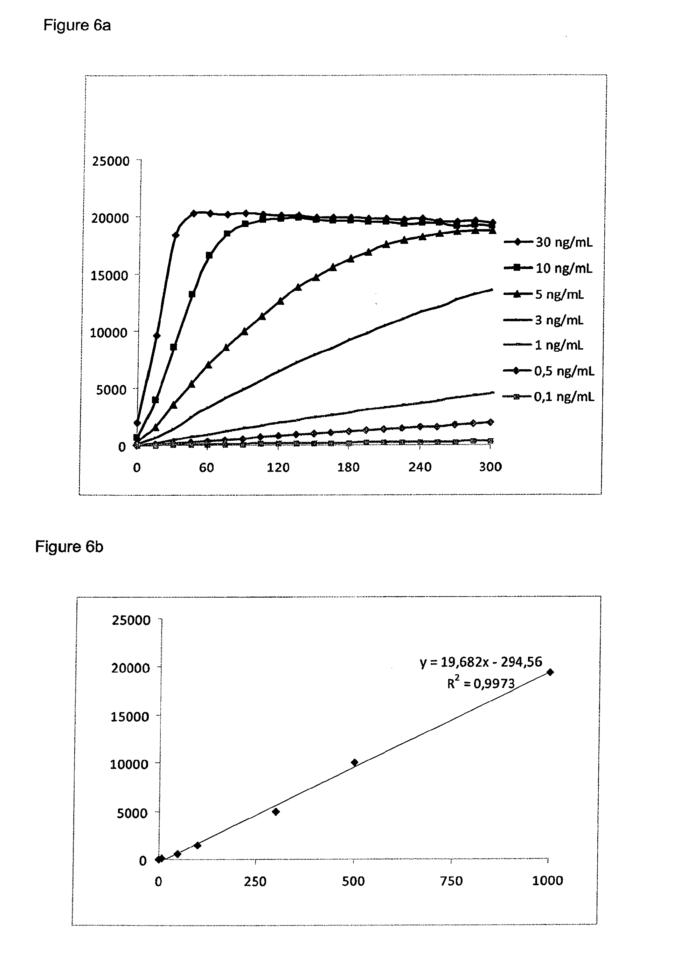

Les différents substrats et leurs métabolites fluorescents sont mis en solution dans le tampon HEPES 50 mM, pH 7,0 aux concentrations respectives de 10⁻⁴ M et 10⁻⁶ M. Les spectres de fluorescence sont enregistrés avec un fluorimètre Perkin Elmer LS50B. Pour les composés de 1 à 12, qui contiennent Pya comme fluorophore, les spectres d'émission obtenus, après excitation à 343 nm, montrent deux maxima à 377 et 397nm (Figure 5), permettant des lectures à plusieurs longueurs d'ondes autour de ces valeurs et λem = 410 nm pour aminobenzoyle (**composé 13**); λem = 420 nm pour Mca.

### Exemple 3 Purification de la Msp

La Msp est purifiée à partir de surnageants de bouillons de culture de 3 litres de *Legionella pneumophila* provenant de la souche Paris selon un protocole basé sur les données initiales de Dreyfus et Iglewski, Infect. Immun., 1986, 51, 736-743.

Le surnageant de culture est d'abord précipité au sulfate d'ammonium 65% sur la nuit à 4°C. Après centrifugation (10 000 rpm, 60 minutes à 4°C), le culot est repris dans environ 200 mL de tampon d'équilibration (25 mM Tris pH 7.8, 25 mM NaCl, 0.01% triton X 100) et dialysé à 4°C pendant une nuit. Le précipité dialysé est ensuite chargé sur une colonne DEAE FF 16/10 (HiPrep, GE Healthcare) à l'aide d'un Akta purifier (GE Healthcare). Les protéines retenues sur la colonne sont éluées sur trois paliers de concentration du tampon d'élution, le premier à 15% de tampon B (25 mM Tris pH 7.8, 1 M NaCl, 0,01% triton X 100), le second à 60 % et le dernier à 100%. L'activité enzymatique correspondante à la Msp est testée dans chaque fraction du second palier à l'aide d'un substrat fluorescent. Les fractions contenant de l'activité enzymatique sont également analysées sur SDS PAGE 10% (BioRad) en conditions non dénaturantes, et les gels colorés au nitrate d'argent (Sigma). Les fractions contenant de l'activité enzymatique Msp sont rassemblées, lavées avec le tampon d'équilibration de la seconde étape de purification, 50 mM Tris pH 7.2, 150 mM NaCl, 0.01% triton X 100 et concentrées sur Centricon YM10 (Amicon). Le « pool » concentré ainsi obtenu est ensuite chargé sur une colonne HiLoad 16/60 Superdex 75 (GE Healthcare) et les protéines éluées avec le tampon d'équilibration à un débit de 0,25 mL/min. Les fractions contenant la Msp sont rassemblées et concentrées après mesure de l'activité enzymatique et électrophorèse des fractions. La pureté de la préparation obtenue est vérifiée sur gel d'électrophorèse. Si nécessaire, cette préparation peut être soumise à une troisième étape de purification sur une colonne de gel filtration Superdex 10/300.

### Exemple 4 Dosage de la Msp par le substrat fluorigénique

Le dosage est effectué sur une préparation de protéase purifiée.

L'essai est effectué en microplaque 96 puits sous un volume final de 100 µL dans le tampon HEPES 50 mM pH 7,0 à 37°C. La Msp est utilisée à 10 ng/mL et le substrat est à une concentration finale de 10 µM. La variation de fluorescence est suivie en continu en fonction du temps dans un lecteur de microplaques Twinkle LB 970 (Berthold) possédant des filtres avec des bandes passantes de ± 15 nm. Pour les substrats de 1 à 12, possédant la pyrénylalanine comme fluorophore, les longueurs d'onde d'excitation et d'émission sont respectivement λex = 340 nm, λem = 405 nm.

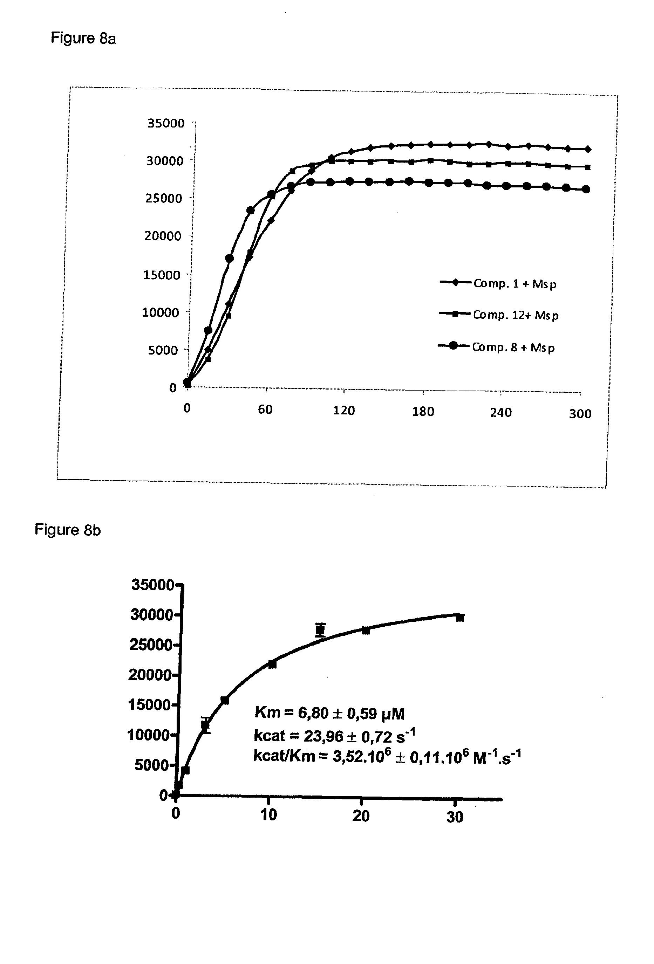

A titre d'exemple, la variation de fluorescence obtenue dans les conditions indiquées ci-dessus avec les substrats **1**, **8 et 12** est présentée sur la Figure 8a.

Les paramètres cinétiques du substrat **1** vis-à-vis de la Msp sont (Figure 8b):
Km = 6,8 ± 0,6 µM kcat = 23,9 ± 0,7 s⁻¹

La sensibilité du substrat **1** a été établie en mesurant la fluorescence émise, en fonction du temps, par hydrolyse d'une concentration donnée du substrat (10 µM) par des quantités de plus en plus faibles de Msp.

### Exemple 5 Relation entre la quantité de Msp détectée dans les surnageants de culture et la quantité de Legionella pneumophila dénombrée dans ces mêmes cultures.

L'essai est effectué en microplaque 96 puits sous un volume final de 100 µL dans le tampon HEPES 50 mM pH 7,0 à 37°C. La Msp est utilisée à 10ng/mL et le substrat est à une concentration finale de 10 µM

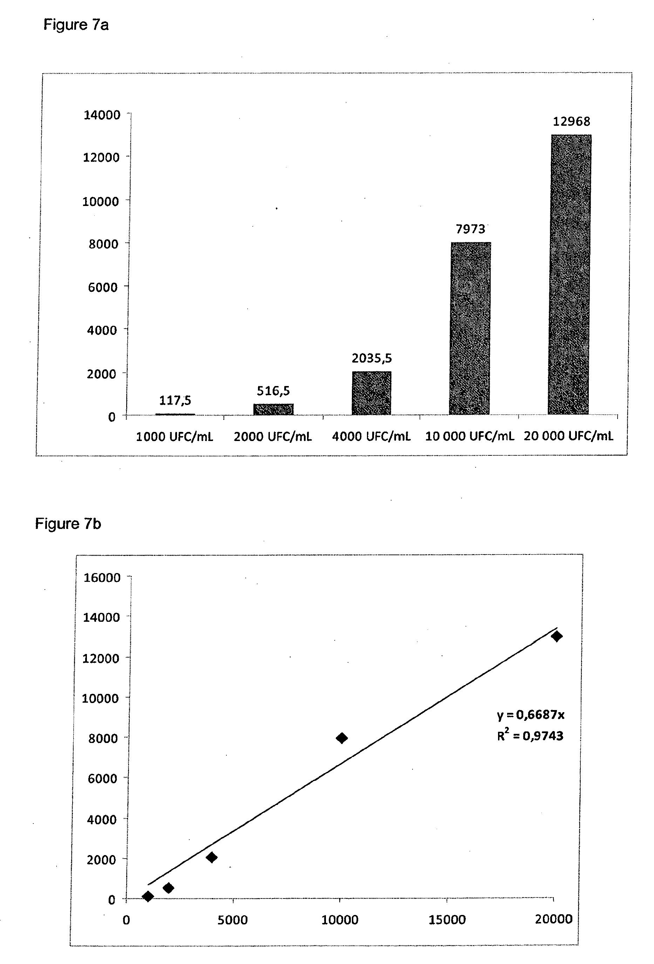

Une première corrélation est établie entre la quantité de Msp présente dans un échantillon et l'intensité de la fluorescence émise pour une quantité déterminée de substrat à différents temps (Figure 7).

Il était ensuite important de vérifier qu'il existait une corrélation entre la quantité de Msp libérée dans un milieu de culture et la quantité de *Legionnella pneumophila* dans ce même milieu.

Ces quantifications ont été établies à partir de différents milieux de culture dont le dénombrement a été effectué suivant le protocole de la norme AFNOR NF R90-431 et la Msp quantifiée à partir du dosage fluorimétrique décrit dans la revendication précédente. On observe en phase stationnaire de croissance une corrélation linéaire entre la quantité de légionelles présentes et la quantité de Msp libérée dans le milieu (Figures 7a et 7b).

## Revendications

1. Peptide substrat sélectif de la Major Secreted Protein (Msp) de *Legionella pneumophila,* de formule (I) :
(I) R-(X)ₙ-Fluo-Rep-(Gly)ₘ-Z-NH₂
dans laquelle,
• Fluo est un acide α-aminé de configuration (L), possédant sur sa chaîne latérale un groupement fluorigénique choisi dans le groupe constitué de la (L)-(1-pyrenyl)-alanine, 1a (L)-Nε(retroAbz)-Lys, la (L)-(7-methoxycoumarin-4-yl)-alanine, la (L)-((6,7-dimethoxy-coumarin-4-yl)-alanine, la (L)-Nβ(pyrenylacetyl)-Dap, la (L)-Nγ(pyrenylacetyl))-Dab, la (L)-Nδ-(pyrenylacetyl)-Orn, la (L)-Nε-(pyrenylacetyl)-Lys, la (L)-S-(1-pyrenemethyl)-Cys et la (L)-O-(1-pyrenemethyl)-Ser,
• Rep est un acide aminé de configuration (L) choisi parmi la (3-NO₂)Tyr et la (4-NO₂)Phe étant entendu que lorsque Fluo est la pyrénylalanine, Rep est la (3-NO₂)Tyr,
• R est un groupement choisi parmi les groupements acétyle, succinyle et méthoxysuccinyle,
• X est un acide α-aminé de configuration (L) choisi dans le groupe constitué par : Gly, Ser, homo-Ser, Lys, homo-Lys, Arg, homo-Arg et Orn,
• Z est un acide α-aminé chargé positivement, et
• n et m sont deux entiers naturels, avec n compris entre 0 et 3 et m compris entre 0 et 2.

2. Peptide selon la revendication 1 **caractérisé en ce que** le reste Fluo est la (L)-(1-pyrenyl)-alanine.

3. Peptide selon l'une quelconque des revendications précédentes **caractérisé en ce que** le reste Rep est la (3-NO₂)Tyr.

4. Peptide selon l'une quelconque des revendications précédentes **caractérisé en ce que** Z est choisi dans le groupe constitué de (L)-Lys, (L)-*homo*-Lys, (L)-Orn, (L)-Arg et (L)-*homo-*Arg.

5. Peptide selon l'une quelconque des revendications précédentes **caractérisé en ce que** ledit peptide est choisi dans le groupe constitué par :
Composé **1-** Ac-Ser-Lys-Gly-**Pya-(3-NO₂)Tyr**-Gly-Gly-Lys-NH₂
Composé **2-** Ac-Ser-Lys-Gly-**Pya-(3-NO₂)Tyr**-Gly-Lys-NH₂
Composé **3-** Ac-Ser-Lys-Gly-**Pya-(3-NO₂)Tyr**-Lys-NH₂
Composé **4-** Ac-Ser-Arg-Gly-**Pya-(3-NO₂)Tyr**-Gly-Gly-Lys-NH₂
Composé **5-** Ac-Arg-Gly-**Pya-(3-NO₂)Tyr**-Gly-Gly-Lys-NH₂
Composé **6-** Ac-Ser-homo-Arg-Gly-**Pya-(3-NO₂)Tyr**-Gly-Gly-Lys-NH₂
Composé **7-** Ac-Ser-Orn-Gly-**Pya-(3-NO₂)Tyr**-Gly-Gly-Lys-NH₂
Composé **8-** Ac-Ser-Lys-Gly-**Pya-(3-NO₂)Tyr**-Orn-NH₂
Composé **9**-Ac-Ser-Lys-Gly-**Pya-(3-NO₂)Tyr**-homo-Lys-NH₂
Composé **10** -Ac-homo-Ser-Lys-Gly-**Pya-(3-NO₂)Tyr**-Orn-NH₂
Composé **11** -Ac-Ser-Lys-Gly-**Pya-(3-NO₂)Tyr**-Arg-NH₂
Composé **12** -Ac-Ser-Lys-Gly-**Pya-(3-NO₂)Tyr**-homo-Arg-NH₂, et
Composé **13** -Ac-Ser-Lys-Gly-**(ε-Abz)Lys-(3-NO₂)Tyr**-Orn-NH₂.

6. Peptide selon la revendication 5 **caractérisé en ce que** ledit peptide est choisi dans le groupe constitué par :
Composé **1-** Ac-Ser-Lys-Gly-**Pya-(3-NO₂)Tyr**-Gly-Gly-Lys-NH₂
Composé **8-** Ac-Ser-Lys-Gly-**Pya-(3-NO₂)Tyr**-Orn-NH₂, et
Composé **12** -Ac-Ser-Lys-Gly-**Pya-(3-NO₂)Tyr**-homoArg-NH₂.

7. Procédé de détection de l'activité protéase de la Major Secreted Protein (Msp) de *Legionella pneumophila* dans un échantillon d'une solution comprenant les étapes de :
a) mettre en contact ledit échantillon avec un composé de formule (I) selon l'une des revendications 1 à 6, et
b) détecter une émission de fluorescence.

8. Procédé selon la revendication 7, **caractérisé en ce que** l'étape a) est réalisée à une température comprise entre 20°C et 55°C, préférentiellement, entre 25°C et 45°C ; plus préférentiellement, entre 30°C et 40°C ; encore plus préférentiellement, entre 35°C et 38°C.

9. Procédé selon la revendication 8, **caractérisée en ce que** la température est égale à 37°C.

10. Procédé de dosage de la Major Secreted Protein (Msp) de *Legionella pneumophila* dans un échantillon d'une solution comprenant les étapes de :
a) détecter l'activité protéase de la Msp selon le procédé des revendications 7 à 9,
b) mesurer l'émission de fluorescence, et
c) déduire de la valeur de l'émission de fluorescence la quantité de Msp présente dans l'échantillon.

11. Procédé de dosage de la Msp selon la revendication 10 **caractérisé en ce qu'**il comporte une étape supplémentaire de concentration de l'enzyme avant l'étape a).

12. Procédé de dosage de la Msp selon la revendication 11 **caractérisé en ce que** la concentration de l'échantillon est réalisée avant l'étape a).

13. Procédé de dosage de *Legionella pneumophila* dans un échantillon d'une solution, comprenant les étapes de :
a) doser la Msp dans ledit échantillon selon le procédé des revendications 10à12,et
b) en déduire la quantité de *Legionella pneumophila.*

14. Procédé selon l'une quelconque des revendications 7 à 13, **caractérisé en ce que** la solution est un milieu susceptible de contenir des bacteries de type *Legionella pneumophila*

15. Procédé selon la revendication 14, **caractérisé en ce que** ladite solution est une eau chaude sanitaire ou une eau de tour aéroréfrigérante.

16. Kit pour la détection et le dosage de *Legionella pneumophila,* ledit kit contenant au moins un peptide selon l'une quelconque des revendications 1 à 6.

## Patentansprüche

1. Peptid, der ein selektives substrat des Sekretierten Hauptproteins (Msp) aus *Legionella Pneumophila* ist, mit der Formel (I):
(I) R(X)ₙ-Fluo-Rep-(Gly)ₘ-Z-NH₂
bei der
Fluo eine (L) Konfigurations-α-Aminosäure ist, die auf ihrer lateralen Kette eine fluorogene Gruppierung besitzt, die ausgewählt ist aus der Gruppe, welche gebildet ist aus (L)-(1-Pyrenyl)-Alamin, (L)-Nε(RetroAbz)-Lys, (L)-7-Methoxykumarin-4-yl)-Alanin, (L)-((6,7-Dimethoxy-Kumarin-4-yl)-Alamin, (L)-Nβ(Pyrenylacetyl)-Dap, (L)-Nγ(Pyrenylacetyl))-Dab, (L)-Nδ-(Pyrenylacetyl)-Orn, (L)-Nε-(Pyrenylacetyl)-Lys, (L)-S-(1-Pyrenemethyl)-Cys und (L)-O-(1-Pyrenemethyl)-Ser,
• Rep eine (L) Konfigurations-Aminosäure ist, die ausgewählt ist aus (3-NO₂)Tyr und (4-NO₂)Phe, wobei es sich von selbst versteht, dass, wenn Fluo Pyrenylalanin ist, Rep (3-NO₂)Tyr ist,
• R eine Gruppierung ist, die ausgewählt ist aus den Gruppierungen Acetyl, Succinyl und Methoxysuccinyl,
• X eine (L) Konfigurations-α-Aminosäure ist, die ausgewählt ist aus der Gruppe, welche gebildet ist aus: Gly, Ser, homo-Ser, Lys, homo-Lys, Arg, homo-Arg und Orn
• Z eine α-Aminosäure ist, die positiv geladen ist, und
• n und m zwei natürliche ganze Zahlen sind, wobei n zwischen 0 und 3 inbegriffen ist und m zwischen 0 und 2 inbegriffen ist.

2. Peptid gemäß Anspruch 1, **dadurch gekennzeichnet, dass** der Fluo-Rest (L)-(1-Pyrenyl)-Alanin ist.

3. Peptid gemäß einem der voranstehenden Ansprüche, **dadurch gekennzeichnet, dass** der Rest Rep (3-NO₂)Tyr ist.

4. Peptid gemäß einem der voranstehenden Ansprüche, **dadurch gekennzeichnet, dass** Z aus der Gruppe ausgewählt ist, die aus (L)-Lys, (L)*-homo*-Lys, (L)-Orn, (L)-Arg und (L)-*homo*-Arg gebildet ist.

5. Peptid gemäß einem der voranstehenden Ansprüche, **dadurch gekennzeichnet, dass** das genannte Peptid ausgewählt ist aus der Gruppe, die gebildet ist aus:
Verbindung **1-** Ac-Ser-Lys-Gly-**Pya-(3-NO₂)Tyr**-Gly-Gly-Lys-NH₂
Verbindung **2-** Ac-Ser-Lys-Gly-**Pya-(3-NO₂)Tyr**-Gly-Lys-NH₂
Verbindung **3-** Ac-Ser-Lys-Gly-**Pya-(3-NO₂)Tyr**-Lys-NH₂
Verbindung **4-** Ac-Ser-Arg-Gly-**Pya-(3-NO₂)Tyr**-Gly-Gly-Lys-NH₂
Verbindung **5-** Ac-Arg-Gly-**Pya-(3-NO₂)Tyr**-Gly-Gyl-Lys-NH₂
Verbindung **6-** Ac-Ser-homo-Arg-Gly-**Pya-(3-NO₂)Tyr-**Gly-Gly-Lys-NH₂
Verbindung **7-** Ac-Ser-Orn-Gly-**Pya-(3-NO₂)Tyr**-Gly-Gly-Lys-NH₂
Verbindung **8-** Ac-Ser-Lys-Gly-**Pya-(3-NO₂)Tyr**-Orn-NH₂
Verbindung **9-** Ac-Ser-Lys-Gly-**Pya-(3-NO₂)Tyr**-homo-Lys-NH₂
Verbindung **10-** Ac-homo-Ser-Lys-Gly-**Pya-(3-NO₂)Tyr-**Orn-NH₂
Verbindung **11-** Ac-Ser-Lys-Gly-**Pya-(3-NO₂)Tyr**-Arg-NH₂
Verbindung **12-** Ac-Ser-Lys-Gly-**Pya-(3-NO₂)Tyr**-homo-Arg-NH₂ und
Verbindung **13-** Ac-Ser-Lys-Gly-**(ε-Abz)Lys-(3-NO₂)Tyr**-Orn-NH₂.

6. Peptid gemäß Anspruch 5, **dadurch gekennzeichnet, dass** das genannte Peptid ausgewählt ist aus der Gruppe, die gebildet ist durch:
Verbindung **1-** Ac-Ser-Lys-Gly-**Pya-(3-NO₂)Tyr**-Gly-Gly-Lys-NH₂
Verbindung **8**- Ac-Ser-Lys-Gly-**Pya-(3-NO₂)Tyr**-Orn-NH₂ und
Verbindung **12**- Ac-Ser-Lys-Gly-**Pya-(3-NO₂)Tyr-**homoArg-NH₂.

7. Detektionsverfahren der Proteaseaktivität des Sekretierten Hauptproteins (Msp) aus *Legionella Pneumophila* in einer Probe einer Lösung, das die Stufen umfasst:
a) die genannte Probe mit einer Verbindung der Formel (I) gemäß Anspruch 1 bis 6 in Kontakt bringen und
b) eine Fluoreszenzemission detektieren.

8. Verfahren gemäß Anspruch 7, **dadurch gekennzeichnet, dass** die Stufe a) bei einer Temperatur realisiert wird, die zwischen 20° C und 55°C, bevorzugt zwischen 25° C und 45° C; stärker bevorzugt zwischen 30° C und 40° C; noch stärker bevorzugt zwischen 35° C und 38° C inbegriffen ist.

9. Verfahren gemäß Anspruch 8, **dadurch gekennzeichnet, dass** die Temperatur gleich 37° C ist.

10. Dosierungsverfahren des Sekretierten Hauptproteins (Msp) von *Legionella Pneumophila* in einer Probe aus einer Lösung, das die Stufen umfasst:
a) Detektieren der Protease-Aktivität der Msp gemäß Anspruch 7 bis 9,
b) Messung der Fluoreszenzemission und
c) Ableitung der in der Probe vorhandenen Msp-Menge aus dem Wert der Fluoreszenzemission.

11. Dosierungsverfahren der Msp gemäß Anspruch 10, **dadurch gekennzeichnet, dass** es eine zusätzliche Konzentrationsstufe des Enzyms vor der Stufe a) umfasst.

12. Dosierungsverfahren der Msp gemäß Anspruch 11, **dadurch gekennzeichnet, dass** die Konzentration der Probe vor der Stufe a) realisiert wird.

13. Dosierungsverfahren von *Legionella Pneumophila* in einer Probe einer Lösung, das die Stufen umfasst:
a) Dosierung der Msp in der genannten Probe gemäß dem Verfahren der Ansprüche 10 bis 12 und
b) daraus Ableitung der Menge an *Legionella Pneumophila.*

14. Verfahren gemäß Anspruch 7 bis 13, **dadurch gekennzeichnet, dass** die Lösung ein Medium ist, das geeignet ist, Bakterien vom Typ *Legionella Pneumophila* zu enthalten.

15. Verfahren gemäß Anspruch 14, **dadurch gekennzeichnet, dass** die genannte Lösung ein warmes Brauchwasser oder ein Wasser aus einem Kühlturm ist.

16. Kit für die Detektion und die Dosierung von *Legionella Pneumophila,* wobei das genannte Kit wenigstens ein Peptid gemäß Anspruch 1 bis 6 enthält.

## Claims

1. Peptide, selective substrate for the Major Secreted Protein (Msp) of *Legionella pneumophila,* of formula (I):
(I) R-(X)ₙ-Fluo-Rep-(Gly)ₘ-Z-NH₂
in which,
Fluo is an α-amino acid of configuration (L), having on its side chain a fluorigenic group, selected from the group consisting of (L)-(1-pyrenyl)-alanine, (L)-Nε(retroAbz)-Lys, (L)-(7-methoxycoumarin-4-yl)-alanine, (L)-((6,7- dimethoxy-coumarin-4-yl)-alanine, (L)-Nß(pyrenylacetyl)-Dap, (L)- Nγ(pyrenylacetyl)) - Dab, (L)-Nδ-(pyrenylacetyl)-Orn, (L)-Nε-(pyrenylacetyl)-Lys, (L)-S-(1-pyrenemethyl)-Cys and (L)-O-(1-pyrenemethyl)-Ser.
• Rep is an amino acid of configuration (L) selected from (3-NO₂)Tyr and (4-NO₂)Phe, with the proviso that when Fluo is pyrenylalanine, Rep is (3-NO₂)Tyr,
• R is a group selected from an acetyl group, a succinyl group and methoxysuccinyl group,
• X is an α-amino acid of configuration (L) selected from the group consisting of: Gly, Ser, homo-Ser, Lys, homo-Lys, Arg, homo-Arg and Orn,
• Z is a positively charged α-amino acid, and
• n and m are two natural whole numbers, with n comprised between 0 and 3 and m comprised between 0 and 2.

2. Peptide according to claim 1 **characterised in that** the Fluo radical is (L)-(1-pyrenyl)-alanine.

3. Peptide according to any of the preceding claims **characterised in that** the Rep radical is (3-NO₂)Tyr.

4. Peptide according to any of the preceding claims **characterised in that** Z is selected from the group consisting of (L)-Lys, *(L)-homo-Lys,* (L)-Orn, (L)-Arg and (*L*)*-homo*-Arg.

5. Peptide according to any of the preceding claims **characterised in that** said peptide is selected from the group consisting of:
Compound **1**- Ac-Ser-Lys-Gly-**Pya-(3-NO₂)Tyr**-Gly-Gly-Lys-NH₂
Compound **2**- Ac-Ser-Lys-Gly-**Pya-(3-NO₂)Tyr**-Gly-Lys-NH₂
Compound **3**- Ac-Ser-Lys-Gly-**Pya-(3-NO₂)Tyr**-Lys-NH₂
Compound **4**- Ac-Ser-Arg-Gly-**Pya-(3-NO₂)Tyr**-Gly-Gly-Lys-NH₂
Compound **5**- Ac-Arg-Gly-**Pya-(3-NO₂)Tyr**-Gly-Gly-Lys-NH₂
Compound **6**- Ac-Ser-homo-Arg-Gly-**Pya-(3-NO₂)Tyr**-Gly-Gly-Lys-NH₂
Compound **7**- Ac-Ser-Orn-Gly-**Pya-(3-NO₂)Tyr**-Gly-Gly-Lys-NH₂
Compound **8**- Ac-Ser-Lys-Gly-**Pya-(3-NO₂)Tyr**-Orn-NH₂
Compound **9**-Ac-Ser-Lys-Gly-**Pya-(3-NO₂)Tyr**-homo-Lys-NH₂
Compound **10** -Ac-homo-Ser-Lys-Gly-**Pya-(3-NO₂)Tyr**-Orn-NH₂
Compound **11** -Ac-Ser-Lys-Gly-**Pya-(3-NO₂)Tyr**-Arg-NH₂
Compound **12** -Ac-Ser-Lys-Gly-**Pya-(3-NO₂)Tyr**-homo-Arg-NH₂, and
Compound **13** -Ac-Ser-Lys-Gly-**(ε-Abz)Lys-(3-NO₂)Tyr**-Orn-NH₂.

6. Peptide according to claim 5 **characterised in that** said peptide is selected from the group consisting of:
Compound **1-** Ac-Ser-Lys-Gly-**Pya-(3-NO₂)Tyr**-Gly-Gly-Lys-NH₂
Compound **8**- Ac-Ser-Lys-Gly-**Pya-(3-NO₂)Tyr**-Orn-NH₂, and
Compound **12**- Ac-Ser-Lys-Gly-**Pya-(3-NO₂)Tyr**-homoArg-NH₂.

7. Method for detecting the protease activity of the Major Secreted Protein (Msp) of *Legionella pneumophila* in a sample of a solution comprising the steps of:
a) contacting said sample with a compound of formula (I) according to one of claims 1 to 6, and
b) detecting a fluorescence emission.

8. Method according to claim 7, **characterised in that** step a) is carried out at a temperature comprised between 20°C and 55°C, preferentially, between 25°C and 45°C; more preferentially, between 30°C and 40°C; even more preferentially, between 35°C and 38°C.

9. Method according to claim 8, **characterised in that** the temperature is equal to 37°C.

10. Method for assaying the Major Secreted Protein (Msp) of *Legionella pneumophila* in a sample of a solution comprising the steps of:
a) detecting the protease activity of Msp according to the method of claims 7 to 9,
b) measuring the fluorescence emission, and
c) deducing from the value of the fluorescence emission the quantity of Msp present in the sample.

11. Method for assaying Msp according to claim 10 **characterised in that** it comprises an additional step of concentrating the enzyme before step a).

12. Method for assaying Msp according to claim 11 **characterised in that** the concentration of the sample is carried out before step a).

13. Method for assaying *Legionella pneumophila* in a sample of a solution, comprising the steps of:
a) assaying the Msp in said sample according to the method of claims 10 to 12, and
b) deducing therefrom the quantity of *Legionella pneumophila.*

14. Method according to any of claims 7 to 13, **characterised in that** the solution is a medium capable of containing bacteria of *Legionella pneumophila* type.

15. Method according to claim 14, **characterised in that** said solution is a domestic hot water or a cooling tower water.

16. Kit for detecting and assaying *Legionella pneumophila,* said kit containing at least one peptide according to any of claims 1 to 6.
